# EUROPEAN PATENT APPLICATION

(11) **EP 4 467 201 A2**
(43) Date of publication of application: **27.11.2024**
(21) Application number: 24206004.4
(22) Date of filing: 05.06.2020
(51) Int. Cl.: A61Q 17/04

(54) **TOPICAL COMPOSITION**

(30) Priority: 07.06.2019 EP 19178934
(62) Divisional of application: 20729794.6
(71) Applicant: DSM IP Assets B.V., 6221 BE Maastricht (NL)
(72) Inventor: DELAUNE, Mathilde, 4303 Kaiseraugst (CH); JANSSEN, Anne, 4303 Kaiseraugst (CH); RADOMSKY, Karina, 4303 Kaiseraugst (CH)
(74) Representative: dsm-firmenich IP

(57) **Abstract**

The present invention relates to topical compositions comprising a saccharide isomerate, a polysiloxane-based UV filter and microfine zinc oxide as well as to the use of a saccharide isomerate to reduce the stickiness of a topical composition comprising a polysiloxane-based UV filter and a microfine zinc oxide.

## Description

The present invention relates to topical compositions comprising a saccharide isomerate, a polysiloxane-based UV filter and microfine zinc oxide as well as to the use of a saccharide isomerate to reduce the stickiness of a topical composition comprising a polysiloxane-based UV filter and a microfine zinc oxide.

It is well known that the ultraviolet (UV) radiation in sun-light has a damaging effect on the skin. Besides the well-known acute damage (sunburn), there is also long-term damage such as an increased risk of developing skin cancer. Moreover, exposure to UV-radiation is responsible for most of the wrinkles and age spots on our faces. Accordingly, to protect the skin against the detrimental effects of UV-light, a range of photoprotective UV-filter substances have been developed that can be incorporated into topical sunscreen preparations.

Modern sunscreens should effectively and safely protect the skin against the harmful effects of UV-radiation. Thus, sunscreens generally contain various UV-B (wavelength: 280-320 nm) and UV-A (wavelength: 320-400 nm) filters. These filters, however, often render the sunscreen sticky, which particularly in beach use means that sand ends up adhering to parts of the skin to which cream has been applied. The greater the UV filter content of a preparation, the greater this problem. To counteract the stickiness problem, consumers tend to use less sunscreen, which however leads to an insufficient protection.

Even though there has been no lack of attempts to develop sunscreen compositions exhibiting a reduced stickiness, this problem has not yet been solved to ultimate satisfaction.

It is therefore an ongoing need to develop novel sunscreen formulations which exhibit a reduced stickiness.

Surprisingly, it has now been found that the addition of a saccharide isomerate to a sunscreen composition comprising a polysiloxane-based UV filter and microfine zinc oxide resulted in a significant reduction of the stickiness.

Thus, in a first aspect, the present invention relates to topical compositions comprising a saccharide isomerate, a polysiloxane-based UV filter and microfine zinc oxide.

The term 'topical' as used herein is understood here to mean external application to keratinous substances, which are in particular the skin, scalp, eyelashes, eyebrows, nails, mucous membranes and hair, preferably the skin of a human.

Saccharide isomerate (CAS 100843-69-4) is a well-known cosmetic agent with unique binding mechanism to the skin used for short and long-lasting moisturization. Saccharide isomerate is e.g. commercially available under the trademark PENTAVITIN^{®} from DSM Nutritional Products Ltd or Hyanify from Lipotec. The most preferred saccharide isomerate in all embodiments of the present invention is PENTAVITIN^{®} which comprises as active ingredient a saccharide isomerate consisting essentially of glucose, fructose mannose and galactose.

The amount of the saccharide isomerate in the topical compositions according to the present invention is preferably in the range from 0.01 to 10 wt.-%, more preferably in the range from 0.1 to 7.5 wt.-%, most preferably in the range from 0.2 to 5 wt.-%, based on the total weight of the topical composition. Further suitable ranges are from 0.25 to 2.5 wt.-% and from 0.5 to 2 wt.-%.

According to a further embodiment, the polysiloxane-based UV filter in the topical composition has a chromophore residue of the benzalmalonate type.

According to a further embodiment, the polysiloxane-based UV filter in the topical composition is a compound according to formula Ia or lb: 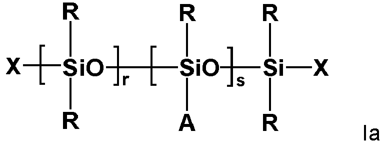 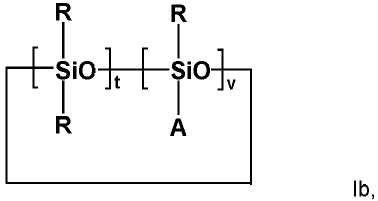 wherein
- X: is R or A;
- A: is selected from formula IIa, IIb or IIc:

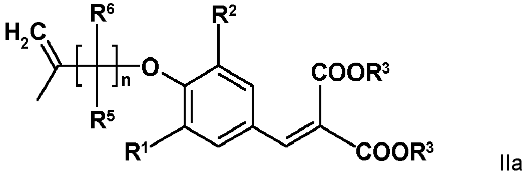

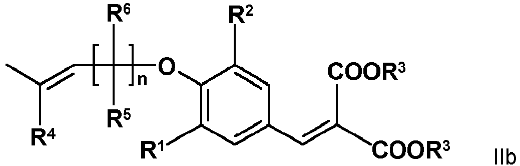

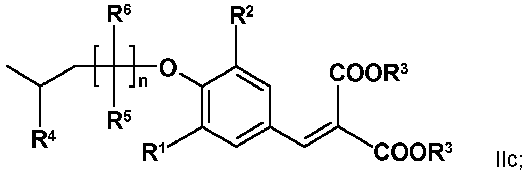

- R: is hydrogen, C₁₋₆-alkyl or phenyl;
- R¹ and R²: are each independently hydrogen, hydroxy, C₁₋₆-alkyl or C₁₋₆-alkoxy;
- R³: is C₁₋₆-alkyl;
- R⁴: is hydrogen or C₁₋₆-alkyl;
- R⁵ and R⁶: are each independently hydrogen or C₁₋₆-alkyl;
- r: is from 0 to 250;
- s: is from 0 to 20;

- r + s: is at least 3;
- t: is from 0 to 10;
- v: is from 0 to 10;
- v + t: is at least 3; and
- n: is from 1 to 6;
with the proviso that when s is 0, at least one X is A.

The term "C₁₋₆-alkyl" refers to groups such as methyl, ethyl, propyl, isopropyl, butyl, sec. butyl, isobutyl, pentyl and neopentyl. The term "C₁₋₆-alkoxy" refers to the corresponding alkoxy groups.

In all embodiments of the present invention, R is preferably methyl.

The residues R¹ and R² are preferably hydrogen, methoxy or ethoxy, more preferably hydrogen, or one of R¹ and R² is hydrogen and the other is methyl, methoxy or ethoxy.

The residues R³ are preferably methyl or ethyl, more preferably ethyl.

Preferably, R⁴ is hydrogen or methyl, R⁵ and R⁶ are hydrogen and n is 1.

The polysiloxane compounds having a group A of the general formula Ila and IIb and their preparation are described in European patent application EP-A0538431. These polysiloxane compounds are most preferred.

The polysiloxane compounds having a group A of the general formula Ilc and their preparation are described in the European patent application EP-A 0358 584.

In the linear polysiloxane compounds according to formula Ia the chromophore carrying residue A may be connected to the end groups of the polysiloxane (X=A) or may be statistically distributed over the polymer.

Linear polysiloxane compounds wherein the chromophore carrying residue A is statistically distributed are preferred. Said preferred polysiloxane compounds have at least one unit carrying the chromophore residue (s = 1), preferably s has a value of from about 2 to about 10, more preferably a statistical mean of about 4. The number of r of the other silicone units present in the polysiloxane compounds is preferably about 5 to about 150, more preferably a statistical mean of about 60.

Polysiloxane compounds wherein 20% or less, preferably less than 10%, of the total siloxane units are units carrying a chromophore residue are preferred with respect to cosmetic properties.

The ratio of polysiloxane units having a chromophore residue A of the formula Ila to those having a chromophore residue A of the formula IIb is not critical. Said ratio may be about 1:1 to about 19:1, preferably about 2:1 to about 9:1, more preferably about 4:1.

The polysiloxane compounds Ia or Ib wherein A is a reside of the formula Ila or IIb can be prepared as described in EP-B0538431 by silylation of the corresponding benzalmalonates according to the following reaction scheme: 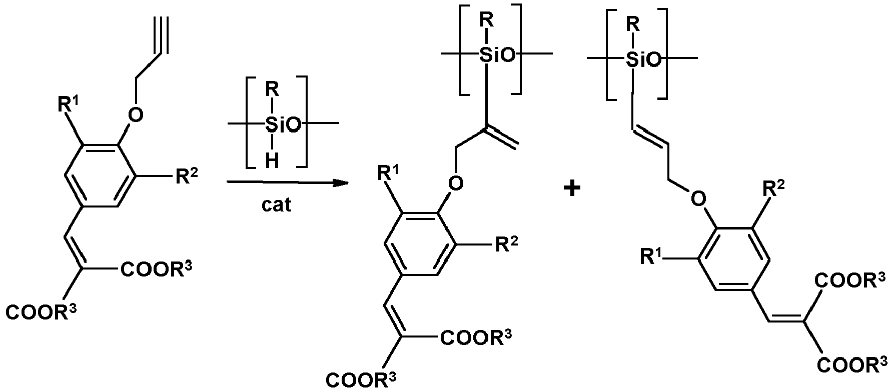 wherein R¹, R² and R³ are as defined above.

The silylation of the 4-(2-propinyloxy)phenyl methylene diethylester may be carried out employing known procedures for the addition of silicon bonded hydrogen atoms to groups containing aliphatic unsaturation. Such reactions are generally catalyzed by a platinum group metal or a complex of such a metal. Examples of catalysts which may be employed are platinum on carbon, chloroplatinic acid, platinum acetyl acetonate, complexes of platinum compounds with unsaturated compounds e.g. olefins and divinyl disiloxanes, complexes of rhodium and palladium compounds and complexes of platinum compounds supported on inorganic substrates. The addition reaction may be performed at reduced, atmospheric or increased pressure. A solvent can be used, e.g. toluene or xylene, in the reaction mixture although the presence of the solvent is not essential. It is also preferred to carry out the reaction at elevated reaction temperatures e.g. from about 50°C to about 150°C.

Particularly preferred are compounds of the general formula la, wherein
- X: signifies methyl,
- A: signifies the group of the formula IIa or IIb,
- R: signifies methyl,
- R¹ and R²: signify hydrogen, methoxy or ethoxy or one or R¹ and R² is hydrogen and the other is methyl, methoxy or ethoxy,
- R³: signifies methyl or ethyl,
- R⁴: signifies hydrogen or methyl,
- R⁵ and R⁶: signify hydrogen,
- r: is about 5 to 150,
- s: is about 2 to about 10, and
- n: has a value of 1.

Most preferred are linear polysiloxanes of the general formula la, wherein
- X: signifies methyl,
- A: signifies a group of the formula IIa or IIb,
- R: signifies methyl,
- R¹ and R²: signify hydrogen,
- R³: signifies ethyl,
- R⁴: signifies hydrogen,
- R⁵ and R⁶: signify hydrogen,
- r: is a statistical mean of about 60,
- s: is a statistical mean of about 4, and
- n: has a value of 1.

These most preferred polysiloxane-based UV filter in all embodiments of the present invention is polysilicone-15 (INCI), which is commercially available under the tradename PARSOL^{®} SLX at DSM Nutritional Products Ltd.

The amount of the polysiloxane-based UV filter and in particular of polysilicone-15 in the compositions according to the present invention is preferably selected in the range 0.1 to 15 wt.-%, such as in the range of 0.5 to 10 wt.-%, such as most preferably in the range of 1 to 5 wt.-% based on the total weight of the composition. Further suitable ranges are 0.2 to 10 wt-%, 0.3-10 wt-% or 0.4 to 10 wt%.

The term microfine zinc oxide refers to any zinc oxide particles suitable for use as UV-filter, i.e. a zinc oxide having a particle size which is principally useful for incorporation into a sunscreen composition. Advantageously the microfine zinc oxide consists of particles having a primary particle size of less than about 300 nm, e.g., less than about 200 nm, or less than about 150 nm. Most preferably, in all embodiments of the present invention the mean particle size of the microfine zinc oxide according to the present invention is selected in the range from 50 to 200 nm, preferably in the range from 75 to 150 nm, most preferably in the range from 90 to 130 nm.

The term 'mean particle size' as used herein refers to the mean number-based particle size distribution Dn50 (also known as Dn0.5) as determined by laser diffraction e.g. with a Horiba particle size distribution analyzer LA-960 or a Malvern Mastersizer 2000 (ISO 13320:2009).

According to the invention, it is also advantageous for zinc oxide particles to be surface-coated. The surface coating may comprise providing the metal oxide particles with a thin hydrophilic or hydrophobic inorganic or organic layer by methods known per se. According to the present invention the different surface coatings can also comprise water. As a result of the surface treatment, the metal oxide is given a hydrophilic, amphiphilic or hydrophobic character.

Examples of inorganic surface coatings which are suitable for the purposes of the instant invention comprise aluminum oxide (Al₂O₃), aluminum hydroxide Al(OH)₃, aluminum oxide hydrate (also: Alumina, CAS-No.: 1333-84-2), sodium hexametaphosphate (NaPO₃)₆, sodium meta-phosphate (NaPO₃)ₙ, silicon dioxide (SiO₂) (also: Silica, CAS-No.: 7631-86-9), and iron oxide (Fe₂O₃). These inorganic surface coatings can be present on their own, in combination and/or in combination with organic coating materials.

Examples of organic surface coatings which are suitable for use in the present invention include vegetable or animal aluminum stearate, vegetable or animal stearic acid, lauric acid, dimethylpolysiloxane (also: dimethicone), methylpolysiloxane (methicone), simethicone (a mixture of dimethylpolysiloxane with an average chain length of about 200 to about 350 dimethylsiloxane units and silica gel), triethoxycaprylylsilane, and octyltrimethoxysilane. These organic surface coatings can be present on their own, in combination and/or in combination with inorganic coating materials. Preferably, the zinc oxide according to the present invention is uncoated or coated with dimethicone, methicone or triethoxycaprylylsilane, most preferably the zinc oxide according to the present invention is surface coated with triethoxycaprylylsilane.

It is furthermore preferred that the zinc oxide is a white powder consisting of zinc oxide present as wurtzite crystal structures.

Zinc oxide particles for use according to the present invention and pre-dispersions of zinc oxide particles are available e.g. available from BASF as Z-Cote or Z-Cote HP1 (2% dimethicone coating), from Tayca as MZ-505S (5% methicone coating), or from DSM Nutritional Products Ltd as PARSOL^{®} ZX (2-3.5% triethoxycaprylylsilane coating).

Most preferably, in all embodiments according to the present invent, the zinc oxide is a white powder consisting of zinc oxide present as wurtzite crystal structures, coated with triethoxycaprylylsilane, which has a zinc oxide content of 96-98%, a triethoxycaprylylsilane content of 2-3.5 % and a mean particle size of 90 to 130 nm.

In all embodiments of the present invention, the amount of the microfine zinc oxide is preferably selected in the range from 0.1 wt.-% to 20 wt.-%, more preferably in the range from 0.5 wt.-% to 15 wt.-%, most preferably in the range from 1 wt.-% to 10 wt.-%, such as in the range from 5 wt.-% to 10 wt.-%, based on the total weight of the topical composition.

In another aspect, the present invention relates to the topical composition according to the embodiments described herein for the use as sunscreen, respectively to the use of the topical composition according to the embodiments described herein as sunscreen.

In yet a further embodiment, the invention relates to the use of saccharide isomerate to reduce the stickiness of a topical composition comprising a polysiloxane-based UV filter and microfine zinc oxide.

A further embodiment of the present invention relates to a method for reducing the stickiness and/ or the sand adherence on skin of a topical composition comprising a polysiloxane-based UV filter and microfine zinc oxide, said method encompassing the step of incorporating saccharide isomerate into said topical composition before applying said composition to skin and optionally appreciating the effect.

As the topical compositions according to the invention are intended for topical application, it is well understood that they comprise a physiologically acceptable medium, i.e. a medium compatible with keratinous substances, such as the skin, mucous membranes, and keratinous fibers. In particular, the physiologically acceptable medium is a cosmetically acceptable carrier.

The term 'cosmetically acceptable carrier' as used herein refers to all carriers and/or excipients and/ or diluents conventionally used in topical cosmetic compositions such as in particular in skin care preparations.

The topical compositions according to the present invention are preferably prepared by admixing the saccharide isomerate, the polysiloxane-based UV filter and the microfine zinc oxide with all the definitions and preferences as given herein with a cosmetically acceptable carrier.

The exact amount of carrier will depend upon the actual level of the saccharide isomerate, the polysiloxane-based UV filter and the microfine zinc oxide and any other optional ingredients that one of ordinary skill in the art would classify as distinct from the carrier (e.g., other active ingredients).

In an advantageous embodiment, the cosmetic or pharmaceutical compositions according to the present invention comprise from 50% to 99%, preferably from 60% to 98%, more preferably from 70% to 98%, such as in particular from 80% to 95% of a carrier, based on the total weight of the topical composition.

In a particular advantageous embodiment, the carrier consists furthermore of at least 40 wt.-%, more preferably of at least 50 wt.-%, most preferably of at least 55 wt.-% of water, such as in particular of 55 to 90 wt.-% of water.

In particular, the topical composition according to the present invention are cosmetic or pharmaceutical compositions, preferably cosmetic (non-therapeutic) compositions.

In one embodiment, the topical compositions according to the present invention are applied to mammalian keratinous tissue such as in particular to human skin or the human scalp and hair.

The term "cosmetic composition" as used in the present application refers to cosmetic compositions as defined under the heading "Kosmetika" in Römpp Lexikon Chemie, 10th edition 1997, Georg Thieme Verlag Stuttgart, New York as well as to cosmetic compositions as disclosed in A. Domsch, "Cosmetic Compositions", Verlag für chemische Industrie (ed. H. Ziolkowsky), 4th edition, 1992.

Preferred topical compositions according to the invention are skin care preparations, decorative preparations, and functional preparations.

Examples of skin care preparations are, in particular, light protective preparations, anti-ageing preparations, preparations for the treatment of photo-ageing, body oils, body lotions, body gels, treatment creams, skin protection ointments, skin powders, moisturizing gels, moisturizing sprays, face and/or body moisturizers, skin-tanning preparations (i.e. compositions for the artificial/sunless tanning and/or browning of human skin), for example self-tanning creams as well as skin lightening preparations.

Examples of decorative preparations are, in particular, lipsticks, eye shadows, mascaras, dry and moist make-up formulations, rouges and/or powders.

Examples of functional preparations are cosmetic or pharmaceutical compositions containing active ingredients such as hormone preparations, vitamin preparations, vegetable extract preparations, anti-ageing preparations, and/or antimicrobial (antibacterial or antifungal) preparations without being limited thereto.

In a particular embodiment, the topical compositions according to the invention are light-protective preparations (sun care products), such as sun protection milks, sun protection lotions, sun protection creams, sun protection oils, sun blocks or tropical's or day care creams with a SPF (sun protection factor). Of particular interest are sun protection creams, sun protection lotions, sun protection milks and sun protection preparations.

The compositions of the invention (including the carrier) may comprise conventional adjuvants and additives, such as preservatives/antioxidants, fatty substances/oils, organic solvents, silicones, thickeners, softeners, emulsifiers, antifoaming agents, aesthetic components such as fragrances, surfactants, fillers, anionic, cationic, nonionic or amphoteric polymers or mixtures thereof, propellants, acidifying or basifying agents, dyes, colorings/colorants, abrasives, absorbents, chelating agents and/ or sequestering agents, essential oils, skin sensates, astringents, pigments or any other ingredients usually formulated into such compositions.

In accordance with the present invention, the compositions according to the invention may also comprise further cosmetically active ingredients conventionally used in cosmetic or pharmaceutical compositions. Exemplary active ingredients encompass UV-filters, agents for the prevention or reduction of inflammation; firming, moisturizing, soothing, and/ or energizing agents as well as agents to improve elasticity and skin barrier.

Examples of cosmetic excipients, diluents, adjuvants, additives as well as active ingredients commonly used in the skin care industry which are suitable for use in the cosmetic compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The necessary amounts of the active ingredients as well as the excipients, diluents, adjuvants, additives etc. can, based on the desired product form and application, easily be determined by the skilled person. The additional ingredients can either be added to the oily phase, the aqueous phase or separately as deemed appropriate.

The cosmetically active ingredients useful herein can in some instances provide more than one benefit or operate via more than one mode of action.

Of course, one skilled in this art will take care to select the above mentioned optional additional ingredients, adjuvants, diluents and additives and/or their amounts such that the advantageous properties intrinsically associated with the combination in accordance with the invention are not, or not substantially, detrimentally affected by the envisaged addition or additions.

The topical compositions according to the present invention may be in the form of a suspension or dispersion in solvents or fatty substances, or alternatively in the form of an emulsion or micro emulsion (in particular of oil-in-water (O/W-) or water-in-oil (W/O-)type, silicone-in-water (Si/W-) or water-in-silicone (W/Si-)type, PIT-emulsion, multiple emulsion (e.g. oil-in-water-in oil (O/W/O-) or water-in-oil-in-water (W/O/W-)type), pickering emulsion, hydrogel, alcoholic gel, lipogel, one- or multiphase solution or vesicular dispersion or other usual forms, which can also be applied by pens, as masks or as sprays.

The topical compositions according to the present invention are advantageously in the form of an oil-in-water (O/W) emulsion comprising an oily phase dispersed in an aqueous phase in the presence of an O/W emulsifier. The preparation of such O/W emulsions is well known to a person skilled in the art and illustrated in the examples.

In one advantageous embodiment, O/W emulsifier is a phosphate ester emulsifier. The term phosphate ester emulsifier refers to phosphate esters emulsifier of formula (II) 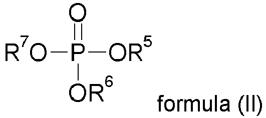 wherein R⁵, R⁶ and R⁷ may be hydrogen, an alkyl of from 1 to 22 carbons, preferably from 12 to 18 carbons; or an alkoxylated alkyl having 1 to 22 carbons, preferably from 12 to 18 carbons, and having 1 or more, preferably from 2 to 25, most preferably 2 to 12, moles ethylene oxide, with the provision that at least one of R⁵, R⁶ and R⁷ is an alkyl or alkoxylated alkyl as previously defined but having at least 6 alkyl carbons in said alkyl or alkoxylated alkyl group.

Monoesters in which R⁵ and R⁶ are hydrogen and R⁷ is selected from alkyl groups of 10 to 18 carbons and alkoxylated fatty alcohols of 10 to 18 carbons and 2 to 12 moles ethylene oxide are preferred. Among the preferred phosphate ester emulsifier are C₈₋₁₀ Alkyl Ethyl Phosphate, C₉₋₁₅ Alkyl Phosphate, Ceteareth-2 Phosphate, Ceteareth-5 Phosphate, Ceteth-8 Phosphate, Ceteth-10 Phosphate, Cetyl Phosphate, C₆₋₁₀ Pareth-4 Phosphate, C₁₂₋₁₅ Pareth-2 Phosphate, C₁₂₋₁₅ Pareth-3 Phosphate, DEA-Ceteareth-2 Phosphate, DEA-Cetyl Phosphate, DEA-Oleth-3 Phosphate, Potassium cetyl phosphate, Deceth-4 Phosphate, Deceth-6 Phosphate and Trilaureth-4 Phosphate. A particular phosphate ester emulsifier according to the invention is potassium cetyl phosphate e.g. commercially available as Amphisol^{®} K at DSM Nutritional Products Ltd Kaiseraugst.

Further suitable O/W emulsifiers according to the present invention encompass PEG-30 Dipolyhydroxystearate, PEG-4 Dilaurate, PEG-8 Dioleate, PEG-40 Sorbitan Peroleate, PEG-7 Glyceryl Cocoate, PEG-20 Almond Glycerides, PEG-25 Hydrogenated Castor Oil, Glyceryl Stearate (and) PEG-100 Stearate , PEG-7 Olivate, PEG-8 Oleate, PEG-8 Laurate, PEG-60 Almond Glycerides, PEG-20 Methyl Glucose Sesquistearate, PEG-40 Stearate, PEG-100 Stearate, PEG-80 Sorbitan Laurate, Steareth-2, Steareth-12, Oleth-2, Ceteth-2, Laureth-4, Oleth-10, Oleth-10/Polyoxyl 10 Oleyl Ether, Ceteth-10, Isosteareth-20, Ceteareth-20, Oleth-20, Steareth-20, Steareth-21, Ceteth-20, Isoceteth-20, Laureth-23, Steareth-100, glycerylstearatcitrate, glycerylstearate (self-emulsifying), stearic acid, salts of stearic acid, polyglyceryl-3-methylglycosedistearate. Further suitable emulsifiers are sorbitan oleate, sorbitan sesquioleate, sorbitan isostearate, sorbitan trioleate, Lauryl Glucoside, Decyl Glucoside, Sodium Stearoyl Glutamate, Sucrose Polystearate and Hydrated Polyisobuten. Furthermore, one or more synthetic polymers may be used as an emulsifier. For example, PVP eicosene copolymer, acrylates/C₁₀₋₃₀ alkyl acrylate crosspolymer, acrylates/steareth-20 methacrylate copolymer, PEG-22/dodecyl glycol copolymer, PEG-45/dodecyl glycol copolymer, and mixtures thereof. Particular preferred emulsifier to be used in the topical compositions according to the present invention are polyhydroxystearic acid, Ceteareth-6 optionally in admixture with stearyl alcohol, Ceteareth-25 as well as mixtures thereof.

Another particular suitable class of O/W emulsifiers are non-ionic self-emulsifying system derived from olive oil e.g. known as (INCI Name) cetearyl olivate and sorbitan olivate (Chemical Composition: sorbitan ester and cetearyl ester of olive oil fatty acids) sold under the tradename OLIVEM 1000.

Further suitable are commercially available polymeric emulsifiers such as hydrophobically modified polyacrylic acid such as Acrylates/C10-30 Alkyl Acrylate Crosspolymers which are commercially available under the tradename Pemulen^{®} TR-1 and TR-2 by Noveon.

Another class of particularly suitable emulsifiers are polyglycerol esters or diesters of fatty acids also called polyglyceryl ester/ diester (i.e. a polymer in which fatty acid(s) is/ are bound by esterification with polyglycerine), such as e.g. commercially available at Evonik as Isolan GPS [INCI Name Polyglyceryl-4 Diisostearate/Polyhydroxystearate/Sebacate (i.e. diester of a mixture of isostearic, polyhydroxystearic and sebacic acids with Polyglycerin-4)] or Dehymuls PGPH available at Cognis (INCI Polyglyceryl-2 Dipolyhydroxystearate).

Also suitable are polyalkylenglycolether such as Brij 72 (Polyoxyethylen(2)stearylether) or Brij 721 (Polyoxyethylene (21) Stearyl Ether e.g. available at Croda.

The at least one O/W respectively Si/W emulsifier is preferably used in an amount of 0.5 to 10 wt.-% such as in particular in the range of 0.5 to 5 wt.-% such as most in particular in the range of 0.5 to 4 wt.-% based on the total weight of the composition.

Suitable W/O- or W/Si-emulsifiers according to the present invention are polyglyceryl-2-dipolyhydroxystearat, PEG-30 dipolyhydroxystearat, cetyl dimethicone copolyol, polyglyceryl-3 diisostearate polyglycerol esters of oleic/isostearic acid, polyglyceryl-6 hexaricinolate, polyglyceryl-4-oleate, polygylceryl-4 oleate/PEG-8 propylene glycol cocoate, magnesium stearate, sodium stearate, potassium laurate, potassium ricinoleate, sodium cocoate, sodium tallowate, potassium castorate, sodium oleate, and mixtures thereof. Further suitable W/Si-emulsifiers are Lauryl Polyglyceryl-3 Polydimethylsiloxyethyl Dimethicone and/or PEG-9 Polydimethylsiloxyethyl Dimethicone and/or Cetyl PEG/PPG-10/1 Dimethicone and/or PEG-12 Dimethicone Crosspolymer and/or PEG/PPG-18/18 Dimethicone. The at least one W/O emulsifier is preferably used in an amount of about 0.001 to 10 wt.-%, more preferably in an amount of 0.2 to 7 wt.-% with respect to the total weigh of the composition.

The topical compositions according to the present invention furthermore advantageously contain at least one co-surfactant such as e.g. selected from the group of mono- and diglycerides and/ or fatty alcohols. The co-surfactant is generally used in an amount selected in the range of 0.1 to 10 wt.-%, such as in particular in the range of 0.5 to 7 wt.-%, such as most in particular in the range of 1 to 5 wt.-%, based on the total weight of the composition. Particular suitable co-surfactants are selected from the list of alkyl alcohols such as cetyl alcohol (Lorol C16, Lanette 16), cetearyl alcohol (Lanette O), stearyl alcohol (Lanette 18), behenyl alcohol (Lanette 22), glyceryl stearate, glyceryl myristate (Estol 3650), hydrogenated coco-glycerides (Lipocire Na10) as well as mixtures thereof

The compositions in form of O/W emulsions according to the invention can be provided, for example, in all the formulation forms for O/W emulsions, for example in the form of serum, milk or cream, and they are prepared according to the usual methods. The compositions which are subject-matters of the invention are intended for topical application and can in particular constitute a dermatological or cosmetic composition, for example intended for protecting human skin against the adverse effects of UV radiation (antiwrinkle, anti-ageing, moisturizing, anti-sun protection and the like).

According to an advantageous embodiment of the invention the compositions constitute cosmetic composition and are intended for topical application to the skin.

Finally, a subject-matter of the invention is a method for the cosmetic treatment of keratinous substances such as in particular the skin, wherein a composition as defined above is applied to the said keratinous substances such as in particular to the skin. The method is in particular suitable to protect the skin against the adverse effects of UV-radiation such as in particular sun-burn and/ or photoageing.

In accordance with the present invention, the compositions according to the invention may comprise further ingredients such as ingredients for skin lightening; tanning prevention; treatment of hyperpigmentation; preventing or reducing acne, wrinkles, lines, atrophy and/or inflammation; chelators and/or sequestrants; anti-cellulites and slimming (e.g. phytanic acid), firming, moisturizing and energizing, self-tanning, soothing, as well as agents to improve elasticity and skin barrier and/or further UV-filter substances and carriers and/or excipients or diluents conventionally used in topical compositions. If nothing else is stated, the excipients, additives, diluents, etc. mentioned in the following are suitable for topical compositions according to the present invention. The necessary amounts of the cosmetic and dermatological adjuvants and additives can, based on the desired product, easily be determined by the skilled person. The additional ingredients can either be added to the oily phase, the aqueous phase or separately as deemed appropriate. The mode of addition can easily be adapted by a person skilled in the art.

The cosmetically active ingredients useful herein can in some instances provide more than one benefit or operate via more than one mode of action.

The topical cosmetic compositions of the invention can also contain usual cosmetic adjuvants and additives, such as preservatives/ antioxidants, fatty substances/ oils, water, organic solvents, silicones, thickeners, softeners, emulsifiers, sunscreens, antifoaming agents, moisturizers, aesthetic components such as fragrances, surfactants, fillers, sequestering agents, anionic, cationic, nonionic or amphoteric polymers or mixtures thereof, propellants, acidifying or basifying agents, dyes, colorings/colorants, abrasives, absorbents, essential oils, skin sensates, astringents, antifoaming agents, pigments or nanopigments, e.g. those suited for providing a photoprotective effect by physically blocking out ultraviolet radiation, or any other ingredients usually formulated into cosmetic compositions. Such cosmetic ingredients commonly used in the skin care industry, which are suitable for use in the compositions of the present invention are e.g. described in the CTFA Cosmetic Ingredient Handbook, Second Edition (1992), The Cosmetic, Toiletry and Fragrance Association, Inc. without being limited thereto.

The necessary amounts of the cosmetic and dermatological adjuvants and additives can - based on the desired product - easily be chosen by a skilled person in this field and will be illustrated in the examples, without being limited hereto.

Of course, one skilled in this art will take care to select the above mentioned optional additional compound or compounds and/or their amounts such that the advantageous properties intrinsically associated with the combination in accordance with the invention are not, or not substantially, detrimentally affected by the envisaged addition or additions.

In one advantageous aspect of the invention, the topical compositions according to the present invention do not contain (i.e. are free of) 3-(4-methylbenzylidene)-camphor, 2-hydroxy-4-methoxybenzophenone (INCI: Oxybenzone), and ethylhexyl 2-cyano-3,3-diphenylacrylate (INCI: Octocrylene).

In another advantageous aspect of the invention, the topical compositions according to the present invention further comprise one or more of dibutyl adipate, dicaprylyl carbonate, C12-C15 alkylbenzoate, Caprylyl Carbonate, Capric/Caprylic Triglyceride as well aa mixtures thereof.

In a still further advantageous aspect of the invention, the topical compositions of the present invention further comprise a preservative and/ or a preservative booster, preferably selected from the group consisting of phenoxyethanol, ethylhexylglycerin, hexylglycerin, glyceryl caprylate, caprylyl glycol, 1,2-hexanediol, propanediol, propylene glycol, p-hydroxyacetophenone as well as mixtures thereof, most preferably selected from the group of p-hydroxyacetophenone and hexyl glycerine as well as mixtures thereof. When present, the preservative respectively the preservative booster is preferably used in an amount of 0.01 to 2 wt.-%, more preferably in an amount of 0.05 to 1.5 wt.-%, most preferably in an amount of 0.1 to 1.0 wt.-%, based on the total weight of the composition.

In another advantageous aspect, the topical compositions according to the present invention are free of any parabenes, benzethoniumchlorid, piroctone olamine, lauroylarginat, methylisothiazolinon, chlormethylisothiazolinon, bronopol, benzalkoniumchloride, formaldeh releasing compounds, salicylic acid, triclosan, DMDM hydantoin, chlorphenesin and IPBC (lodopropinylbutyl carbamate).

In a still further advantageous aspect, the topical compositions of the present invention further comprise one or more of glycerine or polyhydroxystearic acid as well as mixtures thereof.

In yet a still further advantageous aspect, the topical compositions of the present invention further comprise a thickening agent, preferably a gum such as xanthan gum or Caesalpina spinosa gum or a polyacrylate such as polyacrylate crosspolymer-6 as well as mixtures thereof.

The topical compositions according to the invention in general have a pH in the range of 3 to 10, preferably a pH in the range of 4 to 8 and most preferably a pH in the range of 4 to 7. The pH can easily be adjusted as desired with suitable acids such as e.g. citric acid or bases such as NaOH according to standard methods in the art.

The topical compositions according to the invention may further contain one or more emollients which soothe and soften the skin. As an example, the emollient may be dicaprylyl carbonate or C₁₂₋₁₅alkyl benzoate. Further emollients are silicone (dimethicone, cyclomethicone), vegetable oils (grape seed, sesame seed, jojoba, etc.), butters (cocoa butter, shea butter), alcohols (stearyl alcohol, cetyl alcohol), and petrolatum derivatives (petroleum jelly, mineral oil).

The following examples are provided to further illustrate the compositions and effects of the present invention. These examples are illustrative only and are not intended to limit the scope of the invention in any way.

### Example:

All sensorial evaluations have been performed using the emulsion shown in table 1, which were prepared according to standard methods in the art. The determination of the sensorial features was then conducted according to a descriptive sensory evaluation process according to the protocol outlined below:
- Well-trained panellists evaluated the stickiness parameter on a scale from 0-100 compared to references used to calibrate this scale.
- Application was done on the forearm volar surface.
- 50 microliters of the respective emulsion were applied on a spot with an application area of 20 cm² (radius is 2.5cm) corresponding to a topical application of 2.5 mg/cm².
- After application, the respective emulsion was rubbed in until the product was absorbed by the skin.
- The parameter stickiness was evaluated immediately after the rub in phase.
- A statistical analysis was done via p value calculation (ANOVA p-value): If the p-value is less than 0.05 a significant difference does exist.

**Table 1: Formulations (all amounts given in weight-%)**

| | Trade name | INCI | Ref | Ref | Ref | Inv |
|---|---|---|---|---|---|---|
| A | Finsolv TN | C12-15 Alkyl benzoate | 5 | 5 | 5 | 5 |
| | Cetiol CC | Caprylyl Carbonate | 5 | 5 | 5 | 5 |
| | Myritol 318 | Capric/Caprylic Triglyceride | 5 | 5 | 5 | 5 |
| | Dispersun DSP-OL300 | Polyhydroxystearic acid | 1 | 1 | 1 | 1 |
| | Parsol^{®} SLX | Polysilicone-15 | - | - | 3 | 3 |
| B | Parsol^{®} ZX | ZnO, Triethoxy-caprylylsilane | 10 | 10 | 10 | 10 |
| C | Emulgade A6 | Ceteareth-6 & Stearyl Alcohol | 2 | 2 | 2 | 2 |
| | Eumulgin B25 | Ceteareth-25 | 2 | 2 | 2 | 2 |
| | Lanette O | Cetearyl Alcohol | 2 | 2 | 2 | 2 |
| | Lanette 22 | Behenyl Alcohol | 2 | 2 | 2 | 2 |
| D | Symsave H | Hydroxyacetophenone | 0.5 | 0.5 | 0.5 | 0.5 |
| | Solagum TARA | Caesalpina spinosa gum | 0.2 | 0.2 | 0.2 | 0.2 |
| | Sepimax zen | Polyacrylate crosspolymer-6 | 0.5 | 0.5 | 0.5 | 0.5 |
| | Adekanol CHG | Hexyl glycerin | 0.5 | 0.5 | 0.5 | 0.5 |
| | Water | | q.s. | q.s. | q.s. | q.s. |
| | Glycerin | Glycerin | 5 | 5 | 5 | 5 |
| E | Pentavitin | Saccharide Isomerate | - | 1 | - | 1 |
| Stickiness | | | 14.5 | 15.2 | 14.0 | 10.7* |

| | | | | | | |
|---|---|---|---|---|---|---|
| *ANOVA p value 0.046 | | | | | | |

As can be retrieved from the results presented above, a significant reduction of the stickiness was achieved by the addition of saccharide isomerate to a composition comprising PARSOL^{®} SLX and PARSOL^{®} ZX.

## Claims

1. A topical composition comprising a saccharide isomerate, a polysiloxane-based UV filter and microfine zinc oxide.

2. The topical composition according to claim 1, wherein the amount of the saccharide isomerate is selected in the range from 0.01 to 10 wt.-%, preferably in the range from 0.1 to 7.5 wt.-%, most preferably in the range from 0.2 to 5 wt.-%, based on the total weight of the topical composition.

3. The topical composition according to claim 1 or 2, wherein the polysiloxane-based UV filter has a chromophore residue of the benzalmalonate type.

4. The topical composition according to any one of the preceding claims, wherein the polysiloxane-based UV filter is a compound according to formula Ia or lb: 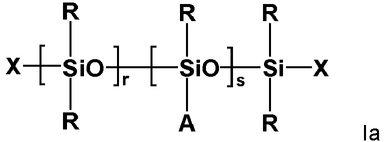 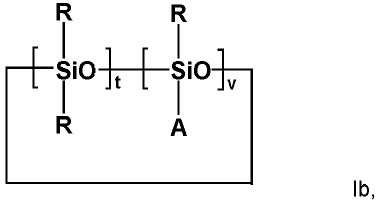 wherein
Xis R or A;
Ais selected from formula Ila, IIb or IIc: 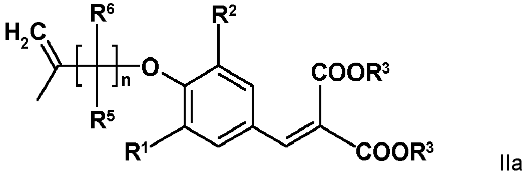 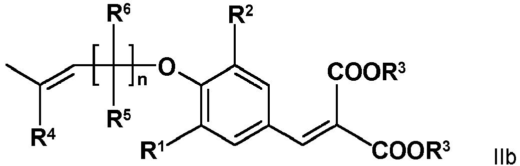 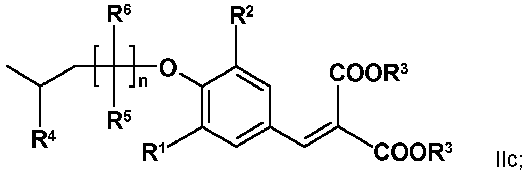
R is hydrogen, C₁₋₆-alkyl or phenyl;
R¹ and R² are each independently hydrogen, hydroxy, C₁₋₆-alkyl or C₁₋₆-alkoxy;
R³ is C₁₋₆-alkyl;
R⁴ is hydrogen or C₁₋₆-alkyl;
R⁵ and R⁶ are each independently hydrogen or C₁₋₆-alkyl;
r is from 0 to 250;
s is from 0 to 20;
r + s is at least 3;
t is from 0 to 10;
v is from 0 to 10;
v + t is at least 3; and
n is from 1 to 6;
with the proviso that when s is 0, at least one X is A.

5. The topical composition according to any one of the preceding claims, wherein the polysiloxane-based UV filter is polysilicone-15.

6. The topical composition according to any one of the preceding claims, wherein the amount of the polysiloxane-based UV filter is selected in the range from 0.1 to 15 wt.-%, preferably in the range from 0.5 to 10 wt.-%, most preferably in the range from 1 to 5 wt.-%, based on the total weight of the composition.

7. The topical composition according to any one of the preceding claims, wherein the microfine zinc oxide has a primary particle size of less than 300 nm, preferably of less than 200nm.

8. The topical composition according to anyone of the preceding claims, wherein the microfine zinc oxide as a mean particle size selected in the range from 50 to 200 nm, preferably in the range from 75 to 150 nm, most preferably in the range from 90 to 130 nm as determined by Laser diffraction.

9. The topical composition according to any one of the preceding claims, wherein the microfine zinc oxide is surface coated, preferably with dimethicone, methicone or triethoxycaprylylsilane, most preferably with triethoxycaprylylsilane.

10. The topical composition according to any one of the preceding claims, wherein the amount of the microfine zinc oxide is selected in the range from 0.1 wt.-% to 20 wt.-%, preferably in the range from 0.5 wt.-% to 10 wt.-%, most preferably in the range of 5 wt.-% to 10 wt.-%, based on the total weight of the composition.

11. The topical composition according to any one of the preceding claims, wherein the composition is in the form of an oil-in-water (O/W) emulsion comprising an oily phase dispersed in an aqueous phase in the presence of an O/W emulsifier.

12. The topical composition according to claim 11, wherein the O/W emulsifier is selected from the group consisting of Ceteareth-25, and Ceteareth-6 & Stearyl Alcohol as well as mixtures thereof.

13. Use of the topical composition according to any one of claims 1 to 12 as sunscreen.

14. Use of saccharide isomerate to reduce the stickiness of a topical composition comprising a polysiloxane-based UV filter and microfine zinc oxide.

15. Method for reducing the stickiness and/ or the sand adherence on skin of a topical composition comprising a polysiloxane-based UV filter and microfine zinc oxide, said method encompassing the step of incorporating saccharide isomerate into said topical composition before applying said composition to skin.
